Europäisches Patentamt

European Patent Office

Office européen des brevets

(11) Veröffentlichungsnummer: **0 236 863**
**A1**

## (12) EUROPÄISCHE PATENTANMELDUNG

(21) Anmeldenummer: **87102737.1**

(22) Anmeldetag: **26.02.87**

(51) Int. Cl.4: **A61B 6/00**

(30) Priorität: **11.03.86 DE 3608069**

(43) Veröffentlichungstag der Anmeldung:
**16.09.87 Patentblatt 87/38**

(84) Benannte Vertragsstaaten:
**DE FR**

(71) Anmelder: **Siemens Aktiengesellschaft Berlin und München**
**Wittelsbacherplatz 2**
**D-8000 München 2(DE)**

(72) Erfinder: **Kranvogel, Feliks**
**Fliederweg 31**
**D-8524 Neunkirchen(DE)**

(54) **Fahrbares Röntgenuntersuchungsgerät.**

(57) Die Erfindung bezieht sich auf ein fahrbares Röntgenuntersuchungsgerät mit einem C-Bogen (1), der an seinen Enden einen Röntgenstrahler (2) und einen Strahlenempfänger (3) trägt, auf einem Wagen (7) verstellbar gelagert und etwa in seiner Mitte an einem Arm (5) angelenkt ist, dessen anderes Ende am Wagen (7) angelenkt ist, wobei die beiden Gelenkachsen (4, 6) parallel zueinander verlaufen. Der Arm weist eine feste Länge auf, wobei diese derart bemessen und der Arm (5) an einer solchen Stelle (6) an dem Wagen (7) angelenkt ist, daß er zum einen in eine etwa senkrechte und zum anderen in eine etwa waagrechte Stellung und der C-Bogen - (1) gleichzeitig jeweils in eine solche Stellung - schwenkbar ist, daß der Röntgenstrahler (2) und der Strahlenempfänger (3) auf einer etwa parallel zu dem Arm (5) verlaufenden Geraden liegen.

FIG 1

EP 0 236 863 A1

## Fahrbares Röntgenuntersuchungsgerät

Die Erfindung betrifft ein fahrbares Röntgenuntersuchungsgerät mit einem C-Bogen, der an seinen Enden einen Röntgenstrahler und einen Strahlenempfänger trägt, auf einem Wagen verstellbar gelagert und etwa in seiner Mitte an einem Arm angelenkt ist, dessen anderes Ende am Wagen angelenkt ist, wobei die beiden Gelenkachsen parallel zueinander verlaufen.

Röntgenuntersuchungsgeräte dieser Art werden für den Operationsraum verwendet. Der C-Bogen muß dabei im Raum vielseitig einstellbar sein, um die Strahlenrichtung den jeweiligen Gegebenheiten entsprechend anpassen zu können.

Ein Röntgenuntersuchungsgerät der eingangs genannten Art ist in der EP-A-0 160 749 beschrieben. Um eine den Erfordernissen entsprechende Einstellbarkeit der Strahlenrichtung zu erreichen, ist der Arm, an dem der C-Bogen angelenkt ist, teleskopartig ausziehbar gestaltet, wodurch der Mechanismus zur Einstellung der Strahlenrichtung kompliziert und dementsprechend teuer und störungsanfällig ist.

Der Erfindung liegt die Aufgabe zugrunde, ein Röntgenuntersuchungsgerät der eingangs genannten Art so auszubilden, daß mit Hilfe eines einfachen Mechanismus eine universelle Einstellung der Strahlenrichtung im Raum möglich ist.

Diese Aufgabe ist erfindungsgemäß dadurch gelöst, daß der Arm eine feste Länge aufweist, wobei diese derart bemessen und der Arm an einer solchen Stelle an dem Wagen an gelenkt ist, daß er zum einen in eine etwa senkrechte und zum anderen in eine etwa waagrechte Stellung und der C-Bogen gleichzeitig jeweils in eine solche Stellung schwenkbar ist, daß der Röntgenstrahler und der Strahlenempfänger auf einer etwa parallel zu dem Arm verlaufenden Geraden liegen. Bei dem erfindungsgemäßen Röntgenuntersuchungsgerät ist somit unter Verzicht auf verstellbare Säulen, Muffen und dergleichen, insbesondere aber unter Verzicht auf einen teleskopartig ausziehbar gestalteten Arm eine universelle Einstellbarkeit des C-Bogens mit Hilfe eines einzigen Armes fester Länge möglich.

Eine besonders zweckmäßige Ausgestaltung ergibt s h, wenn die Kabel für Röntgenstrahler und Strahlenempfänger im C-Bogen und im Arm geführt sind. Bei dieser Ausführung ist auf frei zum Röntgenstrahler und zum Strahlenempfänger verlegte Kabel verzichtet. Die Kabel liegen verdeckt im Arm und im C-Bogen.

Die Erfindung ist nachfolgend anhand eines in der Zeichnung dargestellten Ausführungsbeispieles näher erläutert. Es zeigen:

Fig. 1 ein Röntgenuntersuchungsgerät nach der Erfindung, und

Fig. 2 bis 5 verschiedene Stellungen des Röntgenuntersuchungsgerätes gemäß Figur 1.

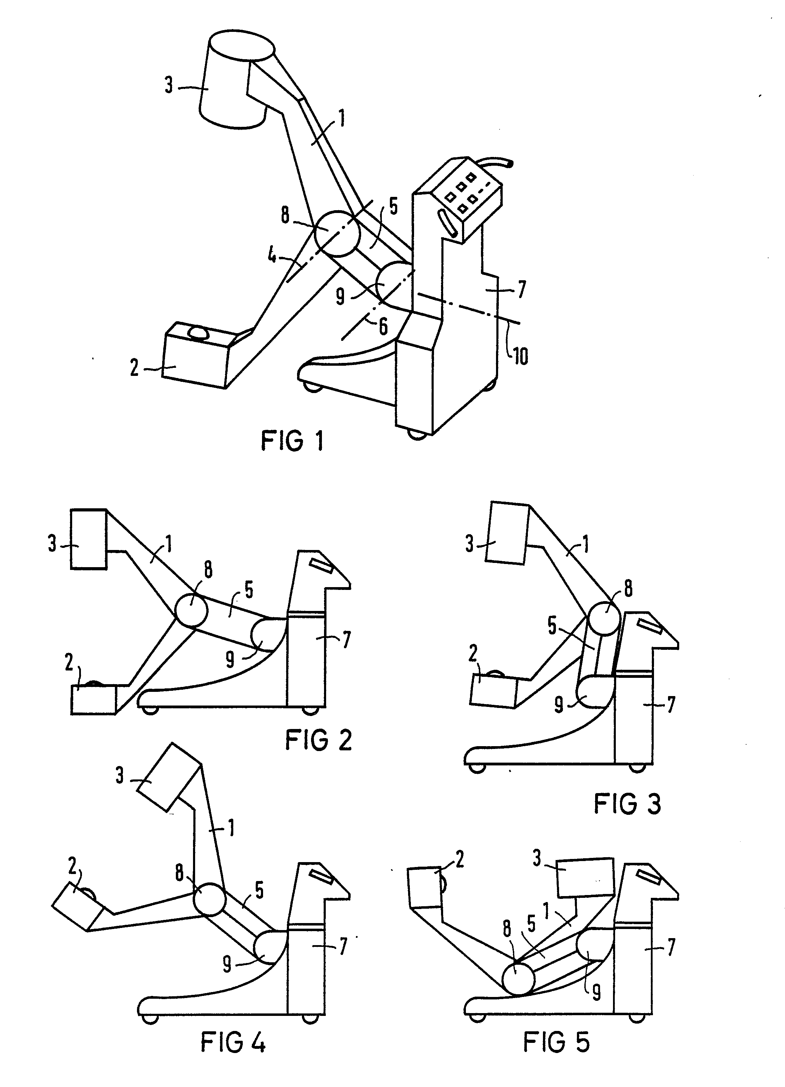

In der Figur 1 ist ein Röntgenuntersuchungsgerät mit einem C-Bogen 1 dargestellt, an dessen Enden ein Röntgenstrahler 2 und ein Röntgenbildverstärker 3 angeordnet sind. Der C-Bogen 1 kann in der Weise an eine Patientenliege herangefahren werden, daß er diese umgreift. Er ist um eine horizontale Achse 4 - schwenkbar mit einem Arm 5 fester Länge verbunden, welcher mit seinem anderen Ende um eine zur Achse 4 parallele, horizontale Achse 6 - schwenkbar an einem Wagen 7 angelenkt ist. Der Wagen 7 ist auf dem Fußboden verfahrbar. Die Verschwenkung des Armes 5 gegenüber dem Wagen 7 um die Achse 6 und die Verschwenkung des etwa in der Mitte gelagerten C-Bogens 1 gegenüber dem Arm 5 um die Achse 4 erfolgt durch Elektromotoren 8, 9.

Die Figuren 2 bis 5 zeigen verschiedene räumliche Lagen des C-Bogens 1. Aus den Figuren 2 bis 5 ergibt sich, daß der Strahlengang im Raum beliebig einstellbar ist. Dies ist trotz der festen Länge des Armes 5 deshalb möglich, weil, wie insbesondere aus den Figuren 3 und 5 ersichtlich ist, unter Berücksichtigung der Gestalt des Wagens 7 die Länge des Armes 5 und die Lage des Gelenkes 6 am Wagen 7 derart gewählt sind, daß der Arm 5 sowohl in eine etwa senkrechte (Fig. 3) als auch in eine etwa waagrechte (Fig. 5) Stellung und der C-Bogen 1 gleichzeitig in eine solche Stellung - schwenkbar ist, daß der Röntgenstrahler 2 und der Strahlenempfänger 3 auf einer etwa parallel zu dem Arm 5 verlaufenden Geraden liegen. Auch Schrägdurchstrahlungen des Patienten sind möglich, da der Arm 5 um eine senkrecht zur Achse 6 und ebenfalls horizontal verlaufende Achse 10 mit dem Wagen 7 drehbar verbunden ist.

Die Kabel zum Röntgenstrahler 2 und zum Röntgenbildverstärker 3 sind im Innern des C-Bogens 1 und des Armes 2 unsichtbar geführt.

### Ansprüche

Fahrbares Röntgenuntersuchungsgerät mit einem C-Bogen (1), der an seinen Enden einen Röntgenstrahler (2) und einen Strahlenempfänger - (3) trägt, auf einem Wagen (7) verstellbar gelagert und etwa in seiner Mitte an einem Arm (5) angelenkt ist, dessen anderes Ende am Wagen (7) angelenkt ist, wobei die beiden Gelenkachsen (4, 6) parallel zueinander verlaufen, **dadurch gekenn-**

zeichnet, daß der Arm (5) eine feste Länge aufweist, wobei diese derart bemessen und der Arm (5) an einer solchen Stelle (6) an dem Wagen (7) angelenkt ist, daß er zum einen in eine etwa senkrechte (Fig. 3) und zum anderen in eine etwa waagrechte (Fig. 5) Stellung und der C-Bogen (1) gleichzeitig jeweils in eine solche Stellung schwenkbar ist, daß der Röntgenstrahler (2) und der Strahlenempfänger (3) auf einer etwa parallel zu dem Arm (5) verlaufenden Geraden liegen.

# 0 236 863

FIG 1

FIG 2

FIG 3

FIG 4

FIG 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 160 749 (SIEMENS AG)<br>* Figur 3; Seite 3, Zeile 19 - Seite 4, Zeile 3; Seite 5, Zeilen 21-30 * | 1 | A 61 B 6/00 |
| | --- | | |
| Y | FR-A-2 405 612 (SIEMENS AG)<br>* Figuren 1,2; Seite 2, Zeile 22 - Seite 3, Zeile 31 * | 1 | |
| | ----- | | |

| | | | RECHERCHIERTE SACHGEBIETE (Int. Cl.4) |
|---|---|---|---|
| | | | A 61 B |

Der vorliegende Recherchenbericht wurde für alle Patentansprüche erstellt.

| Recherchenort | Abschlußdatum der Recherche | Prüfer |
|---|---|---|
| DEN HAAG | 15-06-1987 | CHEN A.H. |